# FASCICULE DE BREVET EUROPEEN

(11) **EP 0 740 507 B1**
(45) Date de publication et mention de la délivrance du brevet: **23.09.1998**
(21) Numéro de dépôt: 95907685.2
(22) Date de dépôt: 20.01.1995
(51) Int. Cl.: A01N 25/28, A61K 7/46

(54) **AMELIORATION DE LA REMANENCE D'UNE ODEUR PAR ENCAPSULATION DU PRINCIPE ODORANT**
VERBESSERUNG DES REMANENZ EINES GERUCHS DURCH VERKAPSELUNG DES RIECHSTOFFES
IMPROVING THE LASTING PROPERTIES OF AN ODOUR BY ENCAPSULATING AN ODORIFEROUS INGREDIENT

(30) Priorité: 20.01.1994 FR 9400579
(43) Date de publication de la demande: 06.11.1996
(73) Titulaire: CAPSULIS, 33600 Pessac (FR)
(72) Inventeur: LAVERSANNE, René, F-33600 Pessac (FR); ROUX, Didier, F-33700 Merignac (FR)
(74) Mandataire: Giraud, Françoise
(86) Numéro de dépôt international: FR9500064
(87) Numéro de publication internationale: WO9519707

(56) Documents cités:
- EP-A- 0 224 457
- EP-A- 0 279 328
- EP-A- 0 307 723
- EP-A- 0 388 239
- EP-A- 0 463 962
- WO-A-85/05009
- WO-A-90/10436
- WO-A-93/19735
- FR-A- 2 233 095
- DATABASE WPI Section Ch, Week 9137 Derwent Publications Ltd., London, GB; Class A12, AN 91-269641 & JP-A-03 146 600 (PROCTER & GAMBLE CO) , 21 Juin 1991

## Description

La présente invention concerne un procédé pour améliorer la rémanence d'une odeur. Elle concerne plus précisément des compositions odorantes à rémanence améliorée ainsi que leurs procédés de préparation et leur mode d'utilisation.

Par produits ou mélanges odorants au sens de l'invention, on entend tous les produits perçus comme tels par l'homme ou les animaux. Ainsi, l'invention concerne aussi bien le domaine des parfums et produits incluant une composition ou un principe odorant dont on cherche à prolonger et à contrôler l'effet olfactif agréable que celui des répulsifs, en particulier des répulsifs pour animaux, où l'on a intérêt à disposer de substances efficaces pendant une période aussi prolongée que possible. Elle concerne également le domaine des phéromones qui sont des substances spécifiquement perçues par certains animaux et dont l'effet sur eux est donc assimilable à celui d'une odeur.

Les répulsifs pour animaux sont des formulations de produits dont l'odeur repousse les animaux. Parmi les produits connus pour avoir cet effet, les extraits de moutarde sont les plus utilisés. Les formulations commerciales présentent l'inconvénient d'avoir un effet très limité dans le temps, et donc nécessitent des applications très fréquentes pour assurer une efficacité permanente (application journalière).

On sait que, dans le domaine des formulations phytosanitaires mais aussi dans celui des compositions odorantes, on a souvent recouru à des mélanges de composés actifs et d'adjuvants ayant pour rôle d'assurer la mise à disposition des actifs. Très souvent le produit actif est une molécule ou un mélange de molécules hydrophobes. Pour des raisons évidentes de facilité de mise en oeuvre il est très utile de formuler le produit actif de façon à le rendre dispersable ou soluble en milieu aqueux. Parmi les méthodes permettant d'atteindre un tel but, l'utilisation de tensioactifs permet souvent une bonne dispersabilité et facilite donc la mise en oeuvre du principe actif.

Une des caractéristiques souvent recherchée dans de telles formulations est la rémanence du principe actif. Une manière de l'obtenir consiste souvent à utiliser un excès de composés. D'autres méthodes ont été utilisées, en particulier dans le domaine phytosanitaire comme l'encapsulation par polymère (de type coacervation par exemple : voir par exemple ANPP deuxième conférence internationale sur les ravageurs en agriculture, "la microencapsulation des produits phytosanitaires", V. Videau et S. Méghir p. 371). D'une façon générale, ces techniques présentent l'inconvénient d'être de mise en oeuvre difficile et onéreuse. De plus, le relargage du principe actif hors de la capsule n'est pas bien contrôlé, et est en général gouverné par la destruction de la capsule, soit chimiquement (hydrolyse), soit mécaniquement.

Indépendamment de l'effet dispersant, les molécules tensioactives permettent dans certains cas de protéger et de vectoriser puis de relarguer de façon contrôlée des molécules actives, ceci, en utilisant des microcapsules formées par une association supramoléculaire des molécules tensioactives. L'exemple le plus courant est celui des liposomes utilisés en cosmétique et dans le domaine bio-médical. Ces liposomes correspondent à un arrangement en vésicules (uni-lamellaires ou multi-lamellaires) de tailles comprises entre quelques centaines d'Angströms et plusieurs microns. Ces vésicules sont, dans le cas des liposomes, obtenus à partir de molécules phospholipidiques (extrait par exemple du soja ou de l'oeuf). Ces liposomes sont capables d'encapsuler des molécules actives lipophiles ou même hydrophiles et ainsi de réaliser les fonctions de vectorisation et de relargage visées.

Le brevet français FR 2 689 418 décrit un procédé qui permet, grâce au recours à une étape de cisaillement d'une phase cristal-liquide lamellaire, de préparer des microcapsules de taille contrôlée, non seulement à partir de tensioactifs lipidiques susceptibles de former des liposomes mais aussi à partir des différents tensioactifs anioniques ou non ioniques et propose l'encapsulation de substances notamment biologiques dans ces capsules.

La demande de brevet européen EP 0 388 239 décrit l'utilisation d'un milieu contenant un tensioactif structuré, formant des microcapsules pour préparer une suspension d'un produit agrochimique insoluble ou très peu soluble dans le milieu. Dans un tel procédé le produit actif agrochimique ne se trouve pas inclus à l'intérieur des microcapsules mais est en suspension dans le milieu entourant ces microcapsules.

La demanderesse a maintenant découvert qu'un moyen pour obtenir un effet olfactif contrôlé et en particulier pour obtenir une rémanence accrue d'une odeur consistait à encapsuler un produit ou une composition odorant dans des microcapsules faites de molécules tensioactives.

Bien que dans le présent mémoire, on utilise les termes "encapsulation" et "microcapsule", ces termes ont des significations sensiblement différentes de celles qu'ils ont lorsqu'il s'agit des microcapsules de type polymérique de l'art antérieur, et cela, du fait de la structure particulière des entités obtenues selon l'invention.

En effet, selon l'invention, c'est le(s) tensioactif(s) qui constitue(nt) les membranes des microcapsules.

Les microcapsules selon l'invention sont plus précisément sous forme multilamellaire, c'est-à-dire qu'elles sont constituées de lamelles concentriques qui leur confèrent une structure du type de celle d'un oignon.

Le produit ou la composition odorant se trouve inclus au sein même de la microcapsule, généralement dans ses membranes, le cas échéant, s'il est purement hydrophile, dans l'eau interstitielle incluse à l'intérieur de la microcapsule. Mais il fait toujours partie intégrante de la microcapsule.

Une telle encapsulation permet d'assurer les fonctions de dispersabilité en milieu aqueux, de vectorisation et de relargage contrôlé du mélange actif responsable de l'odeur.

Elle permet d'améliorer en particulier la rémanence de l'odeur, ce qui s'avère particulièrement utile aussi bien lorsqu'il s'agit de parfumer une atmosphère, une surface, un linge ou un tissu, une personne ou un objet que de répandre une odeur répulsive recherchée tout particulièrement dans le cas des répulsifs pour animaux.

Dans ce dernier cas, tout particulièrement, l'invention permet de formuler un actif agissant comme répulsif pour animal dans des microcapsules et d'obtenir, grâce à cette encapsulation, une activité prolongée du produit actif pouvant dépasser plusieurs semaines après le traitement d'un sol par une dispersion aqueuse de cette formulation.

Un premier avantage de l'invention vient de ce qu'après mise en place du produit formulé, la fuite régulière des capsules assure une mise à disposition lente du principe actif.

Un autre avantage de l'invention réside dans le fait que la fabrication des microcapsules est obtenue directement par mélange du principe actif et d'un mélange judicieusement choisi de tensioactifs.

Un autre avantage vient du fait que l'utilisation des tensioactifs procure une très bonne dispersabilité à la formulation, qui peut être utilisée sous forme liquide, en dispersion aqueuse. Cet aspect est particulièrement avantageux lorsque l'on a affaire à des molécules hydrophobes ou non solubles dans l'eau qui peuvent être dispersées grâce à l'invention sans avoir recours à un solvant organique.

Le mélange se présente, avantageusement sous forme d'un lait, très facilement épandable avec les appareils classiques tels que vaporisateur ou arrosoir, ce qui constitue un autre avantage indéniable de l'invention.

Ainsi selon un premier aspect, l'invention concerne des nouvelles compositions odorantes particulièrement intéressantes pour leur caractère de rémanence mais aussi pour leur forme physique.

Selon un deuxième aspect, l'invention concerne un procédé pour préparer ces compositions.

Selon un troisième aspect, l'invention concerne des utilisations de ces compositions en particulier pour parfumer directement, par exemple par aspersion, une atmosphère, une surface, une personne ou un objet lorsque le principe actif est un parfum agréable. Les compositions de l'invention peuvent également être utilisées comme adjuvants dans des produits lessiviels, détergents ou cosmétiques, en vue d'améliorer la rémanence du parfum qu'ils confèrent.

Les compositions de l'invention peuvent être également utilisées comme agents répulsifs pour les animaux, par exemple pour les animaux domestiques ou les oiseaux lorsque l'on choisit un principe actif ressenti par eux comme malodorant.

Enfin, selon un dernier aspect, l'invention concerne un procédé pour contrôler la diffusion d'une odeur et notamment pour en améliorer la rémanence.

Selon une caractéristique essentielle de son premier aspect, l'invention concerne une composition odorante contenant au moins un agent odorant et au moins un agent tensioactif, dans laquelle ledit agent odorant est encapsulé dans des microcapsules constituées d'un arrangement multilamellaire de bicouches concentriques de tensioactif(s) séparées par un milieu aqueux dit eau interstitielle, ledit agent odorant se trouvant inclus dans les membranes desdites microcapsules lorsqu'il est hydrophobe et/ou dans l'eau interstitielle desdites microcapsules lorsqu'il est hydrophile.

Les microcapsules contenues dans les compositions de l'invention ont avantageusement des dimensions comprises entre 6,1 et 100 µm, de préférence entre 0,2 et 10 µm.

Ces microcapsules peuvent être observées avec un microscope optique. Elles ont avantageusement une taille de préférence de l'ordre du micron. Du fait de cette faible taille, les microcapsules sont soumises à l'agitation brownienne et ne subissent pas ou très peu de décantation ou de crémage.

L'agent ou les agents tensioactifs contenus dans la composition de l'invention peuvent être tout type d'agents tensioactifs susceptibles de former des microcapsules.

Il peut s'agir en particulier de produits lipidiques, en particulier de produits susceptibles de former des liposomes.

Toutefois, on choisira de préférence les agents tensioactifs parmi les tensioactifs non ioniques, anioniques, cationiques ou amphotères.

Ces agents tensioactifs sont avantageusement choisis parmi les dérivés d'acide gras saturés ou insaturés, de préférence parmi les dérivés d'acide gras à chaîne(s) carbonée(s) comprise(s)s entre C₆ et C₂₀ contenant zéro, une ou plusieurs insaturations.

La tête polaire de ces agents tensioactifs peut être ionique de type anionique il s'agira par exemple d'un groupement éthoxylate, sulfate ou sulfonate.

La tête polaire peut également être cationique. il s'agira par exemple d'un ammonium quaternaire, ou d'une amine.

Elle peut également être amphotère. C'est le cas, par exemple de la bétaïne.

La tête peut également être non ionique il pourra s'agir, par exemple, d'un ester ou d'un éther éthoxylé. A titre d'exemple de tels produits on citera les esters de glycérol et de sorbitol, éthoxylés ou non.

La tête polaire peut aussi présenter plusieurs parties, combinant une partie non polaire, comme des segments oxyde d'éthylène et une partie ionique, anionique ou cationique.

Le ou les agents tensioactifs seront avantageusement présents dans la composition à raison de 0,5 à 500 g/l, de préférence de 1 à 200 g/l, de préférence encore de 5 à 100g/l.

Pour la préparation des compositions selon l'invention, on a avantageusement recours à un mélange de tensioactifs choisis soit dans une même classe citée ci-dessus, soit dans des classes différentes.

A titre de mélange de tensioactifs particulièrement utile selon l'invention on citera les mélanges d'oléate et de stéarate de sorbitol et leurs mélanges avec des éthers de sorbitol éthoxylés ou avec des sels d'ammonium ou des sels d'amines.

Un autre mélange tensioactif particulièrement utile selon l'invention est constitué d'un mélange d'alcool gras et d'un tensioactif de type ionique. Un tel mélange peut être en particulier constitué de dodécanol et de dodécylsulfate de sodium.

L'agent odorant pourra être, comme on l'a exposé précédemment, soit un parfum, par exemple une essence ou une composition parfumante. Dans ce cas les microcapsules pourront être utilisées soit à l'état pur sous forme de crème, afin de dispenser sur une longue période le parfum dans l'atmosphère, soit dispersées dans une phase aqueuse, afin d'être vaporisée sur le sol ou un support, ou afin d'imprégner un support poreux. Dans d'autres applications de l'invention, les capsules pourront être avantageusement utilisées soit pour disperser le parfum, par exemple dans des bases de produits détergents, nettoyant pour le sol, lessive, assouplissant, afin de donner une rémanence à l'action parfumante de ces produits, soit introduits dans une préparation cosmétique ou dermocosmétique, par exemple un crème, une émulsion, un gel, un shampooing, afin de bénéficier à la fois de l'avantage de la dispersabilité des microcapsules dans ce type de milieu et de l'effet de rémanence de l'odeur.

L'agent odorant pourra aussi être une mauvaise odeur, soit qu'elle soit perçue comme telle par l'homme, soit qu'elle soit repoussante spécifiquement pour certains animaux, par exemples les canidés ou les félidés, les animaux sauvages nuisibles, les insectes ou les oiseaux. Dans ce cas, la composition sera destinée à être utilisée comme agent répulsif pour ces animaux, afin d'empêcher les dégâts ou les nuisances qu'ils peuvent occasionner.

L'agent odorant pourra aussi être un composé de la classe des phéromones, naturelles ou de synthèse, utilisées pour contrôler le comportement ou la population de certains animaux, mammifères ou insectes. Dans ce cas la composition sera destinée à être utilisée soit dans des pièges, afin d'attirer les animaux, ou à être épandue sur les zones à protéger.

Dans le cas où l'on cherche à éloigner les animaux, le principe actif est avantageusement choisi parmi les composés chimiques présentant une odeur forte, principalement dans la famille des thiols, des amines ou des cétones. Pour cette application, il peut être aussi utilisé des huiles essentielles ou des mélanges de ces huiles qui sont connus pour avoir une action répulsive sur les animaux concernés..

Ce principe actif peut être liquide ou solide à température ambiante.

On choisira de préférence ce principe actif parmi les amines tertiaires et les amines cycliques qui conviennent parfaitement bien.

A titre d'exemples d'autres produits particulièrement adaptés à cette application comme répulsif pour les animaux, on citera, pour les chiens et chats, la pyridine, la 2-undécanone ou la 5-méthyl-2-hexanone et pour les moustiques le diéthyl méta toluiamide (DEET) ou le répulsif 3535 de Merck. A titre d'exemple pour les essences naturelles particulièrement adaptées comme répulsifs, on citera parmi les essences naturelles qui sont connues pour avoir une action répulsive pour les chats, l'essence de rue , pour les moustiques, l'essence de citronnelle ou l'extrait de Neem-Oil, pour les araignées, les extraits de châtaigniers ou de marronniers. La concentration du principe actif est avantageusement comprise entre 0,01 et 250 g/l, de préférence entre 0,1 et 100 g/l, dans le cas des produits répulsifs pour animaux.

Dans le cas où les compositions sont utilisées comme parfum, la teneur minimale pourra bien entendu être nettement plus basse, le seul impératif étant que cette odeur soit perceptible.

Une composition tout particulièrement intéressante selon l'invention et utilisable comme répulsif, notamment pour les chiens et les chats, comprend comme agent tensioactif un mélange d'oléate et de stéarate de sorbitol et comme agent odorant de la pyridine.

Selon un deuxième aspect l'invention concerne un procédé de préparation des compositions décrites ci-dessus.

Le procédé de préparation de l'invention consiste à préparer une phase cristal-liquide ou une suspension de phase cristal-liquide dans l'eau, à solubiliser les molécules actives et à provoquer un réarrangement sous forme de microcapsules des bicouches de tensioactif.

Ce réarrangement peut être selon une première variante effectué spontanément par adjonction d'eau.

Des réarrangements du tensioactif de ce type sont décrits en particulier dans la demande de brevet EP 0 388 239 qui se distingue de la présente invention essentiellement par la nature de la substance active et par le fait qu'elle n'est soluble dans le cas de cette demande ni dans les membranes des microcapsules ni dans l'eau interstitielle, ce qui conduit dans ce cas à l'obtention d'une suspension de produit actif dans un mélange contenant des microcapsules et non comme dans le cas de la présente invention à une dispersion de microcapsules contenant en leur sein le produit actif qui est piégé soit dans la membrane des microcapsules soit dans l'eau interstitielle.

Selon une autre variante de l'invention, le réarrangement des bicouches pour former des microcapsules est provoqué mécaniquement, par cisaillement.

Un tel procédé de formation des microcapsules impliquant une étape de cisaillement est décrit dans la demande de brevet français FR 2 689 418.

On décrit ci-dessous plus précisément un procédé de préparation des compositions de l'invention.

Plus précisément, les microcapsules sont préparées à partir d'une phase cristal-liquide (phase lamellaire par exemple), ou d'une suspension de phase cristal-liquide dans l'eau, dans laquelle sont solubilisées les molécules actives. A partir de cette phase cristal-liquide soit les microcapsules se forment spontanément lors de l'adjonction d'eau soit il est nécessaire d'appliquer un procédé mécanique tel que celui décrit dans le brevet FR 2 689 418 introduit ici par référence. Il est possible de fabriquer ces microcapsules à partir de tensioactifs très variés et non pas seulement de molécules phospholipidiques.

Les capsules correspondent à un arrangement multi-lamellaire de bicouches de tensioactifs séparées par un milieu aqueux. Le produit actif se trouve incorporé soit dans la membrane si il est lipophile soit dans l'eau interstitielle si il est hydrophile. Dans le cas où le produit est partiellement miscible dans un milieu aqueux et organique il se répartit entre la membrane et l'eau piégée. Selon le tensioactif ou le mélange de tensioactifs utilisé, la structure de la membrane est fluide ou à l'état solide (gel).

Une membrane fluide est caractérisée par ce que les molécules tensioactives qui la constituent sont libres de se déplacer dans la surface définie par la bicouche (liquide ou fluide bidimensionnel). Une membrane gel est définie par ce que les molécules qui la constituent forment un réseau solide bidimensionnel. Il est possible de faire passer des membranes de l'état gel à celui de fluide en augmentant la température (transition gel/liquide). Les propriétés de relargage contrôlé sont très différentes selon que la membrane est dans un état fluide ou gel. En particulier dans le cas de l'état gel, la fuite de l'actif est sensiblement ralentie par rapport à l'état fluide. On se placera donc préférentiellement dans le cas où la membrane est dans l'état gel pour formuler des produits à très long temps de rétention.

L'homme du métier sait que le choix des tensioactifs et leurs proportions sont primordiaux, car ils jouent à la fois le rôle d'encapsulant pour le principe actif, et de dispersant pour la formulation. C'est en effet ce choix qui permet de préparer des microcapsules et qui détermine la nature de la membrane qui les compose (gel ou liquide). Les capsules peuvent être faites à partir d'un type ou de plusieurs types de molécules tensioactives. Dans le cas d'un mélange, la proportion de ce mélange permet de régler la température de transition de la membrane de tensioactif (transition gel/liquide), et donc de régler les propriétés encapsulantes des microcapsules.

D'autres composés classiquement utilisés à cette fin peuvent être ajoutés pour améliorer la fluidité de la formulation ou sa dispersabilité.

Pour préparer les compositions de l'invention, on procède avantageusement de la façon suivante : dans un premier temps, on prépare un mélange contenant l'ensemble du ou des tensioactifs, du ou des produits actifs et du minimum d'eau nécessaire pour obtenir une phase homogène. Si nécessaire, ce mélange peut se faire à température élevée (de 10 à 120°C, préférentiellement entre 20 et 70°C). Cette phase est généralement mais, pas obligatoirement, une phase lamellaire cristal-liquide, cette phase cristal-liquide pouvant n'exister qu'à température plus faible que celle utilisée pour solubiliser l'ensemble des constituants. Dans ce cas, il est préférable d'abaisser la température du mélange avant d'effectuer l'étape suivante. La présence d'une phase cristal-liquide lamellaire est facilement caractérisée par une observation sous microscope polarisant (observation de la texture caractéristique de phase lamellaire lyotrope), ou par diffraction des rayonx X qui en plus donne la distance caractéristique entre les membranes dont l'empilement forme la phase lamellaire. La viscosité de cette phase est généralement relativement élevée (de 10⁻² à 10 Pa.s).

Dans l'étape suivante, le mélange initial est dilué avec de l'eau pure sous agitation jusqu'à obtention de la concentration optimale en produits actifs. On peut indépendamment mais, sans obligation, utiliser le procédé décrit dans le brevet FR 2 689 418 afin d'obtenir un ensemble compact de sphérulites de tailles contrôlées. Dans tous les cas, le produit peut être préparé sous forme concentrée puis dilué à la concentration optimum d'utilisation. On peut observer par microscopie optique directement sous microscope les microcapsules formées. Leur taille peut être mesurée en solution diluée par diffusion dynamique de la lumière.

Comme on l'a vu précédemment, l'invention concerne également, selon un troisième aspect, l'utilisation des compositions décrites précédemment ou obtenues selon le procédé décrit précédemment pour parfumer une atmosphère, une surface ou un objet, du linge, une personne, dans le cas où l'agent actif est une substance ou un mélange de parfum agréable, par exemple un extrait, un composé défini ou une base parfumante.

Elle concerne, selon un autre aspect, l'utilisation d'une composition selon l'invention dans laquelle l'agent actif est connu pour son odeur forte ou son action repoussante sur certains animaux comme agent répulsif pour les animaux. Ce principe actif peut être un agent chimique ou une essence naturelle ou un mélange d'essences.

Enfin selon un dernier aspect l'invention concerne, comme on l'a vu précédemment, un procédé pour contrôler la diffusion d'une odeur caractéristique d'un produit ou d'un mélange et notamment pour en augmenter la rémanence, consistant à répandre une composition telle que définie précédemment sur une surface, dans une atmosphère, sur un objet ou par l'intermédiaire d'un produit lessiviel, sur du linge.

Les exemples suivants sont donnés à titre purement illustratif de l'invention.

### Exemple 1

On mélange sous agitation 50 g de pyridine, 15 g d'oléate de sorbitol (Sorban AO commercialisé par Witco) et 20 g de stéarate de sorbitol (Sorban AST commercialisé par Witco). On peut effectuer ce mélange à 60°C afin d'accélérer la dissolution.

Lorsque le mélange est dissous on ajoute sous forte agitation 915 g d'eau pour obtenir 1 l de composé laiteux directement utilisable.

Cette composition répandue sur un trottoir à un taux de 1 g de produit actif (pyridine) par m² conduit à une rémanence de son effet répulsif à l'égard des chiens et des chats de 15 jours.

L'observation en microscopie à contraste de phase de la composition montre qu'elle est constituée d'une dispersion de sphérules dont les dimensions sont comprises entre 1 et 2 µm.

### Exemple 2

La pyridine et les tensioactifs sont mélangés comme dans l'exemple 1 mais en chauffant vers 50°C.

On ajoute dans le mélange 915 g d'eau à 50°C sous agitation modérée et on laisse refroidir lentement le mélange en maintenant l'agitation.

On obtient une composition de structure identique à celle de l'exemple 1 et présentant un effet de rémanence du même ordre.

### Exemple 3

La pyridine et les tensioactifs sont mélangés à chaud comme dans l'exemple 2. Dans le mélange chaud on ajoute lentement 115 g d'eau sous agitation, on laisse refroidir le mélange sous agitation. On obtient une crème fluide, utilisable après dilution dans l'eau, pour obtenir un produit final à la même concentration que celui de l'exemple 1.

Les mêmes effets sont obtenus en ce qui concerne la rémanence de l'effet.

Cette composition répandue sur un trottoir, à un taux de 1 g de produit actif (pyridine) par m² conduit à une rémanence de l'effet répulsif à l'égard des chiens et chats supérieure à 3 semaines.

L'observation en microscopie à contraste de phase de la composition montre qu'elle est constituée d'une dispersion de sphérules de dimensions comprises en 1 et 2µm.

L'effet de ralentissement de la fuite de la pyridine peut être mis en évidence en mesurant la quantité de pyridine restant dans des échantillons préalablement pesés et évaporés dans une atmosphère contrôlée en température et hygrométrie pendant des temps croissants de quelques minutes à quelques semaines. La quantité de pyridine peut être mesurée par chromatographie en phase gazeuse après extraction de l'échantillon évaporé par de l'alcool. Le tracé de la courbe donnant la masse de pyridine restant un fonction du temps, pour un échantillon du produit selon cet exemple , et pour une solution de pyridine dans l'eau montre clairement que dans le cas du produit selon cet exemple, la cinétique d'évaporation de la pyridine à temps long se fait avec une constante de temps de l'ordre de 10 jours, au lieu de 2 h pour la pyridine dans l'eau.

### Exemple 4

Une phase lamellaire est préparée en chauffant à 60°C un mélange de 10 g de polysorbate de stéaryl (Montanox 60, SEPPIC), 42 g de stéarate de sorbitol, de 35 g d'eau et de 12 g de 5-méthyl-2-hexanone, puis en laissant refroidir le mélange sous agitation. La crème obtenue est dispersée dans l'eau pour obtenir une dispersion de microsphères titrée à 2% en cétone.

Cette dispersion peut être utilisée comme répulsif à l'égard des chiens dans les mêmes conditions que le produit de l'exemple 3 avec l'avantage d'avoir une odeur moins forte lors de l'application.

### Exemple 5

La 5-méthyl-2-hexanone de l'exemple 4 peut être remplacée soit par de la ,-undécanone, soit par de l'huile de rue dont la 2-undécanone est l'un des principes odorants. Le mode de préparation est inchangé par rapport à l'exemple 4.

Ce produit est particulièrement efficace à l'égard des chats. Testé dans les mêmes conditions que dans l'exemple 3, il montre une efficacité supérieure à 15 jours.

### Exemple 6

Une dispersion concentrée de microsphères multilamellaires est obtenue en chauffant 10 g de polysorbate de stéaryle (Tween 60, ICI)), 14 g de stéarate de sorbitan (span 60, ICI), 63 g d'eau et 13 g d'extrait de marronnier d'Inde (Flachsmann) sous agitation, jusqu'à 60°C puis en laissant le mélange refroidir en maintenant l'agitation.

Par observation au microscope optique, les microsphères biréfringentes sont bien visibles.

Cette dispersion peut être diluée pour obtenir un produit titré à 4 % d'extrait de marronnier d'Inde. Ce produit pulvérisé sur les sols, murs, poutres etc.. empêche, pour plusieurs semaines, les araignées d'y venir, grâce à la rémanence ainsi conférée à l'action répulsive bien connue des essences de châtaigniers ou marronniers à l'égard de ces animaux.

### Exemple 7

le diéthyl méta toluiamide (DEET) peut être incorporé de la même façon dans des microsphères multilamellaires. Un mélange de 10 g de stéarate de sorbitan, 10 g de polysorbate de stéaryle, 15 g de DEET et 65 g d'eau est chauffé sous agitation jusqu'à 60°C puis refroidi sous agitation. Le mélange ainsi obtenu est formé d'une dispersion de microsphères biréfringentes, et ne montre pas de présence de gouttelettes de DEET émulsionnées.

Le DEET peut être considéré comme une odeur répulsive vis à vis des insectes, en particulier des moustiques. Le produit précédent peut être testé directement sous sa forme concentrée qui a l'aspect et la texture d'une crème cosmétique.

Les tests effectués sur des souris enduites de la composition ci-dessus et mises en présence de moustiques *Aedes Aegypti* en comparaison avec les mêmes tests effectués avec du DEET en solution alcoolique (isopropranol) à la même concentration et avec un produit commercial (Ultrathon) montrent une rémanence accrue de plus d'une heure lorsque le produit est incorporé aux microsphères.

### Exemple 8

### Encapsulation d'essence de lavande

De l'essence de lavande est encapsulée dans les microcapsules de tensioactifs en phase gel.

### a) Préparation du mélange

Un mélange de 12% en poids de dodécylsulfate de sodium, de 18% de dodécanol et de 1% d'essence de lavande est mélangé à 69% d'eau. On obtient une phase cristal-liquide lamellaire en phase gel qui peut être caractérisée par microscopie optique (texture) ou/et par diffraction des rayons X. Les membranes en phase gel à température ambiante se transforment en phase liquide à une température d'environ 40°C, transition que l'on peut observer soit par microscopie optique soit par diffraction des rayons X.

### b) Formation des microcapsules

Pour préparer la phase concentrée de microcapsules on utilise le procédé décrit dans le brevet FR 2 689 418 (application d'un cisaillement homogène de 20 s⁻¹), ceci à une température supérieure à la température de transition gel/liquide. On a choisi T=50°C dans l'exemple décrit. On applique le cisaillement homogène pendant 5 h puis on récupère à température ambiante la crème ainsi formée. Cette crème est composée d'un empilement de microcapsules de l'ordre de 0,4 µm de diamètre. La taille peut être mesurée par diffusion dynamique de la lumière après dispersion des microcapsules dans un large excès d'eau.

Dans la formulation ci-dessus les microcapsules existent spontanément sans l'obligation d'appliquer un cisaillement, cependant l'utilisation du procédé décrit dans le brevet FR 2 689 418 permet d'obtenir une meilleure qualité des microcapsules et un meilleur contrôle de leurs tailles. Ceci assure une meilleure dispersabilité de cette crème dans l'eau.

Que l'on applique ou non le cisaillement, les étapes suivantes sont similaires.

### c) Préparation d'une solution diluée de microcapsules

On peut disperser par agitation cette crème dans un excès d'eau. On obtient ainsi une suspension de microcapsules contenant de l'essence de lavande.

### d) Rémanence de l'odeur

Pour tester l'effet rémanent, on plonge deux morceaux de tissu dans, respectivement, une solution à 1/1 000 de capsules correspondant à 0,01/1 000 d'essence de lavande et dans une solution d'essence de lavande pure (non encapsulée) à 0,01/1 000 dans un mélange hydroalcoolique (afin d'assurer la dissolution complète de l'essence de lavande). Une fois séchée, l'odeur de lavande qui imprègne le tissu persiste après une journée (24 h) pour le tissu plongé dans la solution de microcapsules alors qu'elle disparaît après 2 à 3 h dans le cas de la solution hydroalcoolique.

Une autre manière de tester l'effet de rémanence consiste à disperser dans l'eau, dans plusieurs flacons pouvant être hermétiquement fermés, une quantité de microcapsules telle que la concentration en essence de lavande soit environ 5 pour 1000. Les flacons sont ensuite ouverts pour une période de 10h, 20h, 48h, etc.. puis refermés. L'odeur est ensuite comparée à celle d'une essence de lavande simplement dissoute dans l'eau, à la même concentration et laissés ouverts dans les mêmes conditions. On constate deux phénomènes. Au delà de 24h, les témoins non encapsulés ne sentent plus alors que le parfum lavande est toujours présent dans les échantillons encapsulés au delà de 48h. De plus alors que pour les témoins non encapsulés, le parfum se dénature très rapidement, ne laissant apparaître que les notes de corps en quelques heures, le parfum microencapsulé a gardé son bouquet intact même après 48h d'évaporation.

### Exemple 9

### Parfum microencapsulé pour assouplissant textile

Un mélange composé (en poids) de 5 g de Tween 60 (société ICI), 40 g de span 60 (Société ICI), de 5 g de dialkyl-diméthyl ammonium sur base suif hydrogéné (Noramium M2SH, Société CECA) et de 40 g d'eau est fondu sous agitation à 60°C. Lorsque la température est inférieure à 40°C, 10 g d'une base parfumante pour assouplissant textile (par exemple Vansyn N°1 de Payan-Bertrand, Grasse) sont introduits et l'agitation est maintenue jusqu'à ce que la température revienne à l'ambiante. On obtient une phase lamellaire de texture caractéristique assez fluide. Celle-ci peut être dispersée directement dans une base assouplissante (par exemple 15 % de Déhyquart AU-56 de la Société HENKEL dans l'eau) pour obtenir une concentration en parfum de 0,5 à 1 %

Les essais de rémanence peuvent être effectués soit manuellement par trempage dans l'eau additionnée de l'assouplissant pour le linge ( à raison de 50 ml pour 10 l d'eau), puis séchage à l'air, soit par lavage en machine et séchage au sèche-linge. L'effet de rémanence est très net, le linge restant parfumé plus d'une semaine.

### Exemple 10.

Une composition parfumante rémanente pour nettoyant de sol peut être obtenue en incorporant dans des microsphères multilamellaires un parfum de note "pin".

Les microsphères sont formées d'un mélange de 4,6 g de polysorbate de stéaryl (Tween 60), 16,7 g de stéarate de sorbitol (span 60), de 1,85 g de monoamine éthoxylée (noramox C15, CECA) de 71,3 g d'eau et de 5,5 g de parfum "pin". Après chauffage puis refroidissement sous agitation, on obtient une crème fluide biréfringente qui peut être dispersée dans un produit nettoyant pour le sol.

L'utilisation de ce produit dans les conditions habituelles de nettoyage du sol permet de sentir le parfum "pin" plusieurs heures après le lavage.

### Exemple 11

Des microsphères contenant le parfum "pin" précédent peuvent être dispersées dans l'eau, afin d'obtenir un produit surodorant destiné à être utilisé conjointement au lavage du sol.

La phase lamellaire est obtenu par chauffage sous agitation d'un mélange de 35 g de polysorbate de stéaryl (Tween 60), 5 g de stéarate de sorbitol (span 60), 5 g de chlorure de dialkyl-diméthyl ammonium sur base suif hydrogéné (Noramium M2SH, CECA) 35 g d'eau et 20 g de parfum.

Après refroidissement sous agitation, on obtient une crème épaisse biréfringente qu'on peut disperser très facilement dans l'eau par exemple pour obtenir une dispersion titrée à 1 % de parfum.

Cette dispersion peut être vaporisée directement sur le sol, avant un lavage manuel ou à la machine cireuse. L'odeur de pin reste alors perceptible dans la pièce traitée plus d'une semaine, en particulier sur sols plastiques.

## Revendications

1. Composition odorante contenant au moins un agent odorant et au moins un agent tensioactif caractérisée en ce que ledit agent odorant est encapsulé dans des microcapsules, constituées d'un arrangement multilamellaire de bicouches concentriques de tensioactif(s) séparées par un milieu aqueux dit eau interstitielle, ledit agent odorant se trouvant inclus dans les membranes desdites microcapsules lorsqu'il est hydrophobe et/ou dans l'eau interstitielle desdites microcapsules lorsqu'il est hydrophile.

2. Composition selon la revendication 1, caractérisée en ce que lesdites microcapsules ont des dimensions comprises entre 0,1 et 100 µm, de préférence entre 0,2 et 10 µm.

3. Composition selon l'une des revendications 1 ou 2, caractérisée en ce que la composition contient un tensioactif non ionique, anionique, cationique ou amphotère, ou un mélange de tensioactifs choisis ou non dans la même classe.

4. Composition selon l'une des revendications 1 à 3, caractérisée en ce que le tensioactif est un dérivé d'acide gras saturé ou insaturé.

5. Composition selon la revendication 4, caractérisée en ce que l'acide gras est un acide gras à chaîne(s) carbonée(s) en C₆ à C₂₀ contenant 0, 1 ou plusieurs insaturations.

6. Composition selon l'une des revendications 1 à 5, caractérisée en ce que les microcapsules sont constituées d'au moins un tensioactif de type éthoxylate, sulfate, sulfonate, ester ou éther éthoxylé, par exemple ester de glycérol et de sorbitol éthoxylé ou non, ammonium quaternaire, amine ou bétaïne.

7. Composition selon l'une des revendications 1 à 6, caractérisée en ce qu'elle contient 0,05 à 500 g/l de tensioactif ou de mélange de tensioactifs.

8. Composition selon l'une des revendications 1 à 7, caractérisée en ce que le tensioactif est constitué d'un mélange de stéarate et d'oléate de sorbitol ou d'un mélange de ces composés avec des éthers de sorbitol éthoxylés ou des sels d'ammonium ou d'amines.

9. Composition selon l'une des revendications 1 à 7, caractérisée en ce que le tensioactif est constitué d'un mélange d'alcool gras et d'un tensioactif ionique, par exemple d'un mélange de dodécanol et de dodécylsulfate.

10. Composition selon l'une des revendications 1 à 9, caractérisée en ce que l'agent odorant est un parfum, par exemple une essence ou une composition parfumante.

11. Composition selon l'une des revendications 1 à 10, caractérisée en ce que l'agent odorant est un produit malodorant ou ressenti comme tel par les animaux, le composé étant destiné à être utilisé comme agent répulsif pour animaux.

12. Composition selon la revendication 11, caractérisée en ce que l'agent odorant est un thiol, une amine, de préférence une amine tertiaire ou cyclique telle que la pyridine, la 2-undécanone ou la 5-méthyl-2-hexanone, le diéthyl méta toluiamide (DEET), une essence naturelle connue pour son effet répulsif, telle qu'une essence naturelle connue pour avoir une action répulsive, pour les chats telle que l'essence de rue, pour les moustiques telle que l'essence de citronnelle ou l'extrait de Neem-Oil, pour les araignées telle que les extraits de châtaigniers ou de marronniers.

13. Composition selon l'une des revendications 11 ou 12, caractérisée en ce que le produit odorant est à une concentration comprise entre 0,01 et 250 g/l, de préférence entre 0,1 et 100 g/l.

14. Composition selon l'une des revendications 1 à 10 ,caractérisée en ce que l'agent odorant est une phéromone naturelle ou de synthèse.

15. Composition selon l'une des revendications 1 à 14, caractérisée en ce que l'agent tensioactif est un mélange d'oléate et de stéarate de sorbitol et l'agent odorant est la pyridine.

16. Procédé de préparation d'une composition selon l'une des revendications 1 à 15, caractérisé en ce qu'il consiste à préparer une phase cristal-liquide ou une suspension de phase cristal-liquide et à provoquer un réarrangement des bicouches de tensioactif(s) sous forme de microcapsules.

17. Procédé selon la revendication 16, caractérisé en ce que ledit arrangement est effectué spontanément par adjonction d'eau.

18. Procédé selon la revendication 16, caractérisé en ce que ledit arrangement est provoqué mécaniquement, par cisaillement.

19. Utilisation d'une composition selon l'une des revendications 1 à 15, ou obtenue selon le procédé de l'une des revendications 16 à 18 pour parfumer une atmosphère, une surface, une personne, du linge ou un objet dans le cas où l'agent odorant est une substance de parfum agréable, par exemple une essence.

20. Utilisation d'une composition selon l'une des revendications 1 à 15, ou obtenue selon le procédé de l'une des revendications 16 à 18, dans laquelle l'agent odorant est un agent chimique ou naturel connu pour son odeur désagréable ou ressentie comme telle par les animaux, comme agent répulsif pour animaux.

21. Procédé pour contrôler la diffusion d'une odeur caractéristique d'un produit ou d'un mélange, et notamment pour en augmenter la rémanence, caractérisé en ce qu'il consiste à répandre une composition selon l'une des revendications 1 à 15 ou préparée selon l'une des revendications 16 à 18 sur une surface, dans une atmosphère, sur une personne ou sur un objet ou par l'intermédiaire d'un produit lessiviel sur du linge.

## Claims

1. An odorous composition which contains at least one odorous agent and at least one surfactant agent, characterised in that said odorous agent is encapsulated in microcapsules which are constituted of a multilamellar arrangement of concentric bilayers of surfactant(s) separated by an aqueous medium called interstitial water, said odorous agent being included in the membranes of said microcapsules when it is hydrophobic and/or in the interstitial water of said microcapsules when it is hydrophilic.

2. The composition according to claim 1, characterised in that said microcapsules have dimensions between 0.1 and 100 µm, preferably between 0.2 and 10 µm.

3. The composition according to one of claims 1 or 2, characterised in that the composition contains a non-ionic, anionic, cationic or amphoteric surfactant, or a mixture of surfactants selected from the same class or not.

4. The composition according to one of claims 1 to 3, characterised in that the surfactant is a saturated or unsaturated fatty acid derivative.

5. The composition according to claim 4, characterised in that the fatty acid is a fatty acid with C₆ to C₂₀ carbon chains containing 0, 1 or several unsaturations.

6. The composition according to one of claims 1 to 5, characterised in that the microcapsules are constituted of at least one surfactant of ethoxylate, sulphate, sulphonate, ethoxylated ether or ester type, for example ester of glycerol and of sorbitol, ethoxylated or not, quaternary ammonium, amine or betaine.

7. The composition according to one of claims 1 to 6, characterised in that it contains 0.05 to 500 g/l of surfactant or mixture of surfactants.

8. The composition according to one of claims 1 to 7, characterised in that the surfactant is constituted of a mixture of sorbitol stearate and oleate or a mixture of these compounds with ethoxylated sorbitol ethers or salts of ammonium or of amines.

9. The composition according to one of claims 1 to 7, characterised in that the surfactant is constituted of a mixture of fatty alcohol and an ionic surfactant, for example a mixture of dodecanol and dodecyl sulphate.

10. The composition according to one of claims 1 to 9, characterised in that the odorous agent is a perfume, for example an essence or a perfuming composition.

11. The composition according to one of claims 1 to 10, characterised in that the odorous agent is a malodorous product or sensed as such by animals, the compound being intended to be used as an animal repellent agent.

12. The composition according to claim 11, characterised in that the odorous agent is a thiol, an amine, preferably a tertiary or cyclic amine such as pyridine, 2-undecanone or 5-methyl-2-hexanone, diethyl meta toluiamide (DEET), a natural essence known for its repellent effect, such as a natural essence known to have a repellent action for cats, such as essence of rue, for mosquitoes, such as essence of citronella or extract of Neem-oil, for spiders, such as extracts of chestnuts or horse chestnuts.

13. The composition according to one of claims 11 or 12, characterised in that the odorous product is at a concentration between 0.01 and 250 g/l, preferably between 0.1 and 100 g/l.

14. The composition according to one of claims 1 to 10, characterised in that the odorous agent is a natural or synthetic pheromone.

15. The composition according to one of claims 1 to 14, characterised in that the surfactant agent is a mixture of sorbitol oleate and stearate and the odorous agent is pyridine.

16. A method of preparing a composition according to one of claims 1 to 15, characterised in that it consists in preparing a liquid-crystal phase or a suspension of liquid-crystal phase and in causing a rearrangement of the bilayers of surfactant(s) in the form of microcapsules.

17. The method according to claim 16, characterised in that said arrangement is effected spontaneously by the addition of water.

18. The method according to claim 16, characterised in that said arrangement is caused mechanically by shearing.

19. Use of a composition according to one of claims 1 to 15, or one obtained according to the method of one of claims 16 to 18, for perfuming an atmosphere, a surface, a person, linen or an object in the case of the odorous agent being a substance of pleasant perfume, for example an essence.

20. Use of a composition according to one of claims 1 to 15, or one obtained according to the method of one of claims 16 to 18, in which the odorous agent is a chemical or natural agent known for its unpleasant odour or sensed as such by animals, as an animal repellent agent.

21. A method for controlling the diffusion of a characteristic odour of a product or a mixture, and particularly for increasing the lasting properties thereof, characterised in that it consists in spreading a composition according to one of claims 1 to 15, or prepared according to one of claims 16 to 18, on a surface, in an atmosphere, on a person or on an object or via a clothes washing product.

## Patentansprüche

1. Geruchs-Zusammensetzung, die mindestens ein riechendes bzw. duftendes Agens und mindestens ein oberflächenaktives Agens enthält, dadurch gekennzeichnet, daß das riechende bzw. duftende Agens in Mikrokapseln eingekapselt ist, die aus einer multilamellaren Anordnung von konzentrischen Doppelschichten aus dem (den) oberflächenaktiven Agens (Agentien) bestehen, die durch ein als interstitielles Wasser bezeichnetes wäßriges Medium voneinander getrennt sind, wobei das genannte riechende bzw. duftende Agens in den Membranen der genannten Mikrokapseln eingeschlossen ist, wenn es hydrophob ist, und/oder in dem interstitiellen Wasser der genannten Mikrokapseln eingeschlossen ist, wenn es hydrophil ist.

2. Zusammensetzung nach Anspruch 1, dadurch gekennzeichnet, daß die genannten Mikrokapseln Dimensionen zwischen 0,1 und 100 µm, vorzugsweise zwischen 0,2 und 10 µm, haben.

3. Zusammensetzung nach einem der Ansprüche 1 oder 2, dadurch gekennzeichnet, daß sie ein nicht-ionisches, anionisches, kationisches oder amphoteres oberflächenaktives Agens oder ein Gemisch von oberflächenaktiven Agentien, ausgewählt aus solchen der gleichen Klasse oder nicht der gleichen Klasse, enthält.

4. Zusammensetzung nach einem der Ansprüche 1 bis 3, dadurch gekennzeichnet, daß das oberflächenaktive Agens ein Derivat einer gesättigten oder ungesättigten Fettsäure ist.

5. Zusammensetzung nach Anspruch 4, dadurch gekennzeichnet, daß die Fettsäure eine Fettsäure mit C₆-C₂₀-Kohlenstoffketten ist, die 0, 1 oder mehr Unsättigungen enthält.

6. Zusammensetzung nach einem der Ansprüche 1 bis 5, dadurch gekennzeichnet, daß die Mikrokapseln aus mindestens einem oberflächenaktiven Agens vom Ethoxylat-, Sulfat-, Sulfonat-, ethoxylierten Ester- oder ethoxylierten Ether-Typ, beispielsweise vom ethoxylierten oder nicht-ethoxylierten Glycerin- und Sorbit-Ester-Typ, vom quaternären Ammonium-, Amin- oder Betain-Typ aufgebaut sind.

7. Zusammensetzung nach einem der Ansprüche 1 bis 6, dadurch gekennzeichnet, daß sie 0,05 bis 500 g/l eines oberflächenaktiven Agens oder eines Gemisches von oberflächenaktiven Agentien enthält.

8. Zusammensetzung nach einem der Ansprüche 1 bis 7, dadurch gekennzeichnet, daß das oberflächenaktive Agens aus einem Gemisch von Sorbitstearat und Sorbitoleat oder einem Gemisch dieser Verbindungen mit ethoxylierten Sorbitethern oder Ammonium- oder Aminsalzen besteht.

9. Zusammensetzung nach einem der Ansprüche 1 bis 7, dadurch gekennzeichnet, daß das oberflächenaktive Agens aus einem Gemisch von Fettalkohol und einem ionischen oberflächenaktiven Agens, beispielsweise einem Gemisch von Dodecanol und Dodecylsulfat besteht.

10. Zusammensetzung nach einem der Ansprüche 1 bis 9, dadurch gekennzeichnet, daß das riechende bzw. duftende Agens ein Parfüm, beispielsweise ein etherisches Öl (eine Essenz) oder eine wohlriechende Zusammensetzung (Parfüm-Zusammensetzung) ist.

11. Zusammensetzung nach einem der Ansprüche 1 bis 10, dadurch gekennzeichnet, daß das riechende bzw. duftende Agens ein übelriechendes Produkt ist oder von Tieren als ein solches empfunden wird, wenn die Zusammensetzung als Mittel zur Abwehr (Abstoßung) von Tieren verwendet werden soll.

12. Zusammensetzung nach Anspruch 11, dadurch gekennzeichnet, daß das duftende bzw. riechende Agens ein Thiol, ein Amin, vorzugsweise ein tertiäres oder cyclisches Amin wie Pyridin, 2-Undecanon oder 5-Methyl-2-hexanon, Diethyl-m-toluylamid (DEET), ein für seine abstoßende Wirkung bekanntes natürliches ätherisches Öl (Essenz), z.B. ein natürliches ätherisches Öl, das bekannt dafür ist, daß es eine abstoßende Wirkung hat auf Katzen, wie Rautenöl, auf Stechmücken (Moskitos), wie Zitronenöl oder Neem-Öl-Extrakt, auf Spinnen, wie die Extrakte von Kastanienbäumen oder Edelkastanienbäumen.

13. Zusammensetzung nach einem der Ansprüche 11 oder 12, dadurch gekennzeichnet, daß der Duftstoff in einer Konzentration zwischen 0,01 und 250 g/l, vorzugsweise zwischen 0,1 und 100 g/l, vorliegt.

14. Zusammensetzung nach einem der Ansprüche 1 bis 10, dadurch gekennzeichnet, daß der Duftstoff ein natürliches oder synthetisches Pheromon ist.

15. Zusammensetzung nach einem der Ansprüche 1 bis 14, dadurch gekennzeichnet, daß das oberflächenaktive Agens ein Gemisch aus Sorbitoleat und Sorbitstearat ist und daß der Duftstoff Pyridin ist.

16. Verfahren zur Herstellung einer Zusammensetzung nach einem der Ansprüche 1 bis 15, dadurch gekennzeichnet, daß man eine Flüssigkristall-Phase oder eine Flüssigkristall-PhasenSuspension herstellt und eine Umlagerung der Doppelschichten aus einem oder mehreren oberflächenaktiven Agentien in Form von Mikrokapseln hervorruft.

17. Verfahren nach Anspruch 16, dadurch gekennzeichnet, daß die genannte Umlagerung spontan bewirkt wird durch Zugabe von Wasser.

18. Verfahren nach Anspruch 16, dadurch gekennzeichnet, daß die genannten Umlagerung auf mechanischem Wege durch Scheren hervorgerufen wird.

19. Verwendung einer Zusammensetzung nach einem der Ansprüche 1 bis 15 oder einer solchen, wie sie bei dem Verfahren nach einem der Ansprüche 16 bis 18 erhalten wird, zum Parfümieren einer Atmosphäre, einer Oberfläche, einer Person, von Wäsche oder eines Gegenstands (Formkörpers) für den Fall, daß der Duftstoff eine Substanz mit angenehmem Geruch, beispielsweise ein ätherisches Öl (eine Essenz), ist.

20. Verwendung einer Zusammensetzung nach einem der Ansprüche 1 bis 15 oder einer solchen, wie sie bei dem Verfahren nach einem der Ansprüche 16 bis 18 erhalten wird, als Mittel zur Abwehr (Abstoßung) von Tieren, in der der Duftstoff ein chemisches oder natürliches Agens ist, das bekannt ist für seinen unangenehmen oder von Tieren als ein solcher empfundenen Geruch.

21. Verfahren zur Steuerung (Kontrolle) der Diffusion eines charakteristischen Geruches eines Produkts oder einer Mischung, insbesondere zur Erhöhung der Remanenz desselben (derselben), dadurch gekennzeichnet, daß man eine Zusammensetzung nach einem der Ansprüche 1 bis 15 oder eine solche, die nach dem Verfahren nach einem der Ansprüche 16 bis 18 hergestellt worden ist, auf einer Oberfläche, in einer Atmosphäre, auf einer Person oder auf einem Gegenstand (Formkörper) oder mittels eines Waschmittels auf der Wäsche verteilt (ausbreitet).
